## Europäisches Patentamt
## European Patent Office
## Office européen des brevets

(19)

(11) Publication number: **0 112 012**
— A2

(12) **EUROPEAN PATENT APPLICATION**

(21) Application number: **83306655.8**

(22) Date of filing: **01.11.83**

(51) Int. Cl.³: **C 12 N 15/00**
C 12 P 21/02, C 12 N 1/20
//C12R1/19

(30) Priority: **08.11.82 US 439683**

(43) Date of publication of application:
**27.06.84 Bulletin 84/26**

(84) Designated Contracting States:
**BE CH DE FR GB IT LI NL SE**

(71) Applicant: **Case Western Reserve University**
**Cleveland Ohio 44106(US)**

(72) Inventor: **Rottman, Fritz Max**
**28500 Gates Mills Blvd.**
**Pepper Pike Ohio(US)**

(72) Inventor: **Woychik, Richard P.**
**2585 Euclid Heights Blvd. No. 3**
**Cleveland Heights Ohio(US)**

(72) Inventor: **Nilson, John Helmer**
**2324 Canterbury Road**
**University Heights Ohio(US)**

(74) Representative: **Perry, Robert Edward et al,**
**GILL JENNINGS & EVERY 53-64 Chancery Lane**
**London WC2A 1HN(GB)**

(54) Genomic bovine growth hormone, its preparation, and hosts containing it.

(57) A bovine growth hormone genomic clone has been prepared by use of recombinant DNA techniques. The nucleotide sequence of this genomic clone, and functional equivalents thereof, can be cloned into suitable vectors for the transformation into micro-organism hosts which can then be cultured to produce bovine growth hormone. Novel modified hosts and plasmids are described.

EP 0 112 012 A2

### GENOMIC BOVINE GROWTH HORMONE, ITS PREPARATION, AND HOSTS CONTAINING IT

This invention relates to genomic bovine growth hormone, its preparation, and hosts containing it.

Bovine growth hormone (BGH) is a 191 amino-acid poly-peptide, synthesised initially as a pregrowth hormone containing a signal peptide of 26 amino-acids; see Hunt et al, "Atlas of Protein Sequence and Structure", ed. M.O. Dayhoff (National Biomedical Research Foundation, Washington, D.C.) Vol. 5, Suppl. 2, pp. 113-139, and Lingappa et al, Proc. Natl. Acad. Sci. 74 (1977) 2432-2436. The 3'-untranslated region of BGH has been shown to be 104 nucleotides in length; see Sadavage et al, Biochemistry 19 (1980) 1737-1743. It has been shown that the RNA of bovine proclactin (PRL) and BGH comprise most of the mRNA population of the bovine anterior pituitary; see Nelson et al, J. Biol. Chem. 254 (1979) 1516-1520.

Miller et al, J. Biol. Chem. 255 (1980) 7521-7524, and Keshet et al, Nucleic Acids Res. 9 (1981), have reported the cloning of BGH cDNA.

EP-A-0067026 discloses the cloning of bovine pituitary growth hormone cDNA into the Pst 1 site of plasmid pBR322 by the oligo(dG)-oligo(dC) tailing technique.

A BGH genomic clone according to the invention, designated pλGH2R2, was deposited in the permanent collection of the Northern Regional Research Laboratory (NRRL), U.S. Department of Agriculture, Peoria, Illinois, U.S.A. It was deposited on 21st November 1982 in E. coli HB101 as host, and its accession number in this repository is NRRL B-15154. On 22nd November 1982, in the same host, the genomic clone pλGH2R3 was deposited; its accession number is NRRL B-15155.

The nucleotide sequence of genomic BGH of the present invention is as follows:-

AAAACCTATGGGGTGGGCTCTCAAGCTGAGACCCTGTGTGTGCACAGCCCTCTGGCTGGTGGCAGTGGAG

ACGGGATNNNATGACAAGCCTGGGGGGACATGACCCCAGAGAAGGAACGGGAACAGGATGAGTGAGAGGAG

GTTCTAAATTATCCATTAGCACAGGCTGCCAGTGGTCCTTGCATAAATGTATAGAGCACACAGGTGGGGG

                       5'

GAAAGGGAGAGAGAGAAGAAGCCAGGGTATAAAAATGGCCCAGCAGGGACCAATTCCAGGATCCCAGG⊤:

                    -26
                    Met Met Ala Ala

CCAGTTCACCAGACGACTCAGGGTCCTGTGGACAGCTCACCAGCT ATG ATG GCT GCA G gtaag

ctcgctaaaatcccctccattcgcgtgtcctaaaggggtaatgcggggggggccctgccgatggatgtgttc

agagctttgggctttagggcttccgaatgtgaacataggtatctacacccagacatttggccaagtttga

aatgttctcagtccctggagggaagggtaggtgggggctggcaggagatcaggcgtctagctccctgggg

                  -20
                 Gly Pro Arg Thr Ser Leu Leu Leu Ala

ccctccgtcgcggccctcctggtctctccctag  GC CCC CGG ACC TCC CTG CTC CTG GCT

       -10                  1
Phe Ala Leu Leu Cys Leu Pro Trp Thr Gln Val Val Gly Ala Phe Pro Ala——
TTC GCC CTG CTC TGC CTG CCC TGG ACT CAG GTG GTG GGC GCC TTC CCA GCC

          10                    20
Met Ser Leu Ser Gly Leu Phe Ala Asn Ala Val Leu Arg Ala Gln His Leu
ATG TCC TTG TCC GGC CTG TTT GCC AAC GCT GTG CTC CGG GCT CAG CAC CTG

                 30
His Gln Leu Ala Ala Asp Thr Phe Lys Glu Phe
CAT CAG CTG GCT GCT GAC ACC TTC AAA GAG TTT   gtaagctcccgaggggatgcgt

cctaggggtggggaggcaggaaggggtgaatccacacccctccacacagtgggaggaaactgaggagtt

cagccgtattttatccaagtagggatgtggttaggggagcagaaacggggggtgtgtggggtggggagggt

tccgaataaggcggggaggggaaccgcgcaccagcttagacctgggtgggtgtgttcttcccccag

                 40
Glu Arg Thr Tyr Ile Pro Glu Gly Gln Arg Tyr Ser Ile Gln Asn Thr Gln
GAG CGC ACC TAC ATC CCG GAG GGA CAG AGA TAC TCC ATC CAG AAC ACC CAG

50                   60
Val Ala Phe Cys Phe Ser Glu Thr Ile Pro Ala Pro Thr Gly Lys Asn Glu
GTT GCC TTC TGC TTC TCT GAA ACC ATC CCG GCC CCC ACG GGC AAG AAT GAG

      70
Ala Gln Gln Lys Ser
GCC CAG CAG AAA TCA   gtgagtggcaacctcggaccgaggagcaggggacctccttcatcctaa

gtaggctgccccagctctccgcaccgggcctggggcggccttctccccgaggtggcggaggttgttggat

ggcagtggaggatgatggtgggcggtggtggcaggaggtcctcgggcagaggccgaccttgcagggctgc

                     Asp Leu Glu Leu Leu Arg Ile
cccaagcccgcggcacccaccgaccacccatctgccagcag GAC TTG GAG CTG CTT CGC ATC

```
                80                                          90
Ser Leu Leu Leu Ile Gln Ser Trp Leu Gly Pro Leu Gln Phe Leu Ser Arg
TCA CTG CTC CTC ATC CAG TCG TGG CTT GGG CCC CTG CAG TTC CTC AGC AGA


                   100                                          110
Val Phe Thr Asn Ser Leu Val Phe Gly Thr Ser Asp Arg Val Tyr Glu Lys
GTC TTC ACC AAC AGC TTG GTG TTT GGC ACC TCG GAC CGT GTC TAT GAG AAG


                   120
Leu Lys Asp Leu Glu Glu Gly Ile Leu Ala Leu Met Arg
CTG AAG GAC CTG GAG GAA GGC ATC CTG GCC CTG ATG CGG gtggggatggcgt

tgtgggtcccttccatgctggggggccatgcccgccctctcctggcttagccaggagaatgcacgt

gggcttggggagacagatccctgctctctccctctttctagcagtccagccttgacccaggggaa

accttttcccctttttgaaacctccttcctcgcccttctccaagcctgtaggggagggtggaaaat

ggagcgggcaggagggagctgctcctgagggcccttcggcctctctgtctctccctcccttggcag

                   130                                          140
Glu Leu Glu Asp Gly Thr Pro Arg Ala Gly Gln Ile Leu Lys Gln Thr
GAG CTG GAA GAT GGC ACC CCC CGG GCT GGG CAG ATC CTC AAG CAG ACC


                   150
Tyr Asp Lys Phe Asp Thr Asn Met Arg Ser Asp Asp Ala Leu Leu Lys
TAT GAC AAA TTT GAC ACA AAC ATG CGC AGT GAC GAC GCG CTG CTC AAG


        160                                          170
Asn Tyr Gly Leu Leu Ser Cys Phe Arg Lys Asp Leu His Lys Thr Glu
AAC TAC GGT CTG CTC TCC TGC TTC CGG AAG GAC CTG CAT AAG ACG GAG


                   180                                          190
Thr Tyr Leu Arg Val Met Lys Cys Arg Arg Phe Gly Glu Ala Ser Cys
ACG TAC CTG AGG GTC ATG AAG TGC CGC CGC TTC GGG GAG GCC AGC TGT


Ala Phe am
GCC TTC TAG  TTGCCAGCCATCTGTTGTTTGCCCCTCCCCCGTGCCTTCCTTGACCCTG


                                                          +3'
GAAGGTGCCACTCCCACTGTCCTTTCCTAATAAAATGAGGAAATTGCATCGC ATTGTCTG

AGTAGGTGTCATTCTATTCTGGGGGGGTGGGGTGGGGCAGGACAGCAAGGGGGAGGATTGGGA

AGACAATAGCAGGCATGCTGGGGATGCGGTGGGCTCTATGGGTACCCAGGTGCTGAAGAA

TTGACCCGGTTCCTCCTGGG
```

Note:  Lower case letters correspond to intron sequences.

The subject invention includes the above nucleotide sequence and functional equivalents of the nucleotide sequence.  By "functional equivalents" is meant nucleotide sequences which can be used in substantially the same way to get substantially the same results in the preparation of bovine growth hormone by a suitable host.

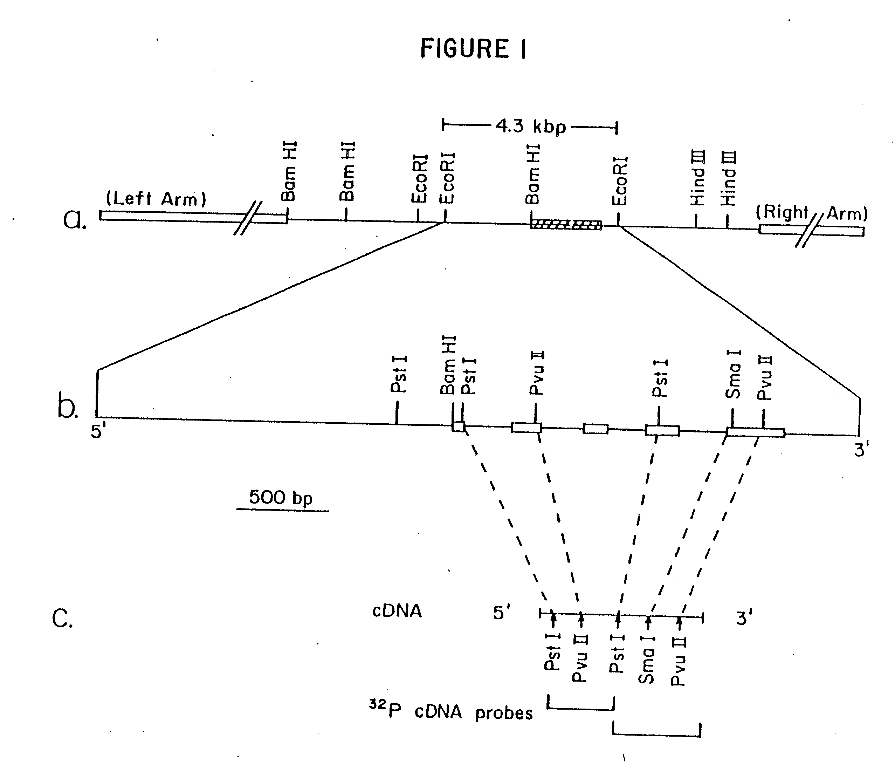

In the accompanying drawings, Figure 1 shows the structure of the bovine growth hormone genomic and cDNA clones. Part (a) shows the structure of the genomic λGh2. The hatched box represents the growth hormone gene. Part (b) shows the restriction map of the 4.3 kbp EcoRI fragment from λGh2 that was subcloned in pBR322. Boxed regions represent the exon regions on the gene. Part (c) shows the structure of the insert sequence from the growth hormone cDNA clone, pLG23, which is disclosed in EP-A-0067026.

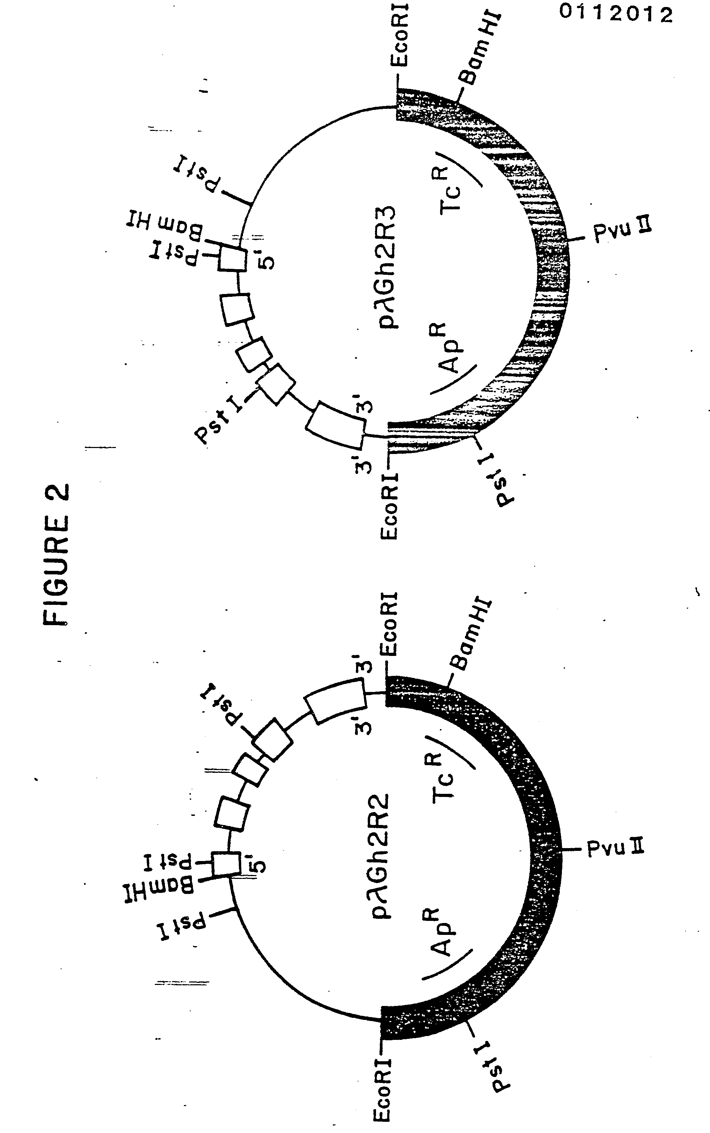

Figure 2 shows the structure of the pλGh2R2 and the pλGh2R3 genomic subclones. For orientation purposes, the tetracycline and ampicillin resistance genes, as well as a few restriction enzyme sites, are shown on the pBR322 DNA portion (shown in solid black). The open boxes represent the exon regions in the growth hormone gene sequence.

BGH genomic clone pλGH2R2, NRRL B-15154, contains the entire bovine growth hormone gene as well as several hundred nucleotides of 5' and 3' flanking sequence. The given nucleotide sequence of the bovine growth hormone gene shows that it is approximately 1.8 kbp (kilobase pairs) in length and contains five exons and four intervening sequences. The 5' flanking region contains a TATA box 88 base pairs upstream from the first AUG; see Gannon et al, Nature 278 (1979) 428-434.

The first exon is primarily 5' untranslated sequence and contains only four amino-acid codons. The four intervening sequences are all roughly the same size and contain the characteristics "GT" and "AG" dinucleotides at their 5' and 3' boundaries; see Breathnach et al, Proc. Natl. Acad. Sci. USA, 75 (1978) 4853-4857.

There is an AATAAA sequence on the 3' untranslated region located 18 nucleotides upstream from the polyadenylation site. This hexanucleotide is found in the 3' untranslated regions of most eukaryotic genes; see Proudfoot et al, Nature 263 (1976) 211-214. The nucleotide has been shown to be involved in the polyadenylation reaction (Fitzgerald, J., and Shenk, T. (1981) Cell 24, 251-260).

The utility of the BGH genomic clones of the subject invention is readily apparent to a person skilled in the art. They can be used to direct the synthesis of BGH in a suitable eukaryotic host, e.g., cell lines and organisms. Such hosts are well known to persons skilled in the art. Also, the clones can be used to make genomic BGH nucleotide sequence which can be used to clone into other vectors and hosts by methods well known in the art.

The above disclosure with the possession of the genomic clones enables a person skilled in the art to practice the claimed invention. However, to foster a better understanding of this art, there follows a disclosure of procedures employed to prepare the key genomic clones of the subject invention.

Example 1    Genomic DNA Preparation

An adult bovine pituitary (2.2 g) was obtained and was immediately frozen in liquid nitrogen. The tissue was ground to a powder in a chilled mortar and pestle and was then immediately digested with 100 µg/ml proteinase K (EM Biochemicals) at 50°C in 0.01 M Tris-HCl pH 8, 0.1 M NaCl, 0.01 M EDTA and 0.5% SDS. When all the tissue was dissolved (approximately 4 hours), the sample was extracted three times with Tris-saturated phenol and three times with $CHCl_3$, then ethanol precipitated. The precipitated material was slowly resuspended over the course of several days at 4°C in 0.01 M Tris-HCl (pH=8.0) and 0.001 M EDTA. The DNA concentration was estimated using the diphenylamine technique (Burton, K. (1968) in Methods in Enzymology (L. Grossman and K. Moldave, eds.) Vol. 12, part B, pp. 163-166. Academic press, New York). Using agarose gel electrophoresis, the size of the DNA was estimated to be greater than 80 kbp.

Placental DNA was a gift from Dr. Les Hoffman (University of Wisconsin). 6.5 g of frozen bovine placental tissue was placed in liquid nitrogen, powdered and then immediately digested with 100 µg/ml proteinase K (Beckman) in 0.1 M EDTA and 1% sarkosyl at 55°C. The solution was extracted with a 1:1 mixture of NETS (0.1 M NaCl, 0.005 M EDTA, 0.01 M Tris-HCl, pH 7.5 and 0.5% SDS)-saturated phenol: CIAA (CIAA is $CHCl_3$:isoamyl alcohol 24:1) followed by two extractions in CIAA. The nucleic acids were ethanol precipitated and the DNA was spooled around a pasteur pipette. The DNA was dissolved in 0.01 M Tris-HCl, pH 7.5, 0.001 M EDTA and was subjected to equilibrium cesium chloride centrifugation. The banded DNA was collected from the gradient and dialyzed in 0.01 M Tris-HCl, pH 7.5 and 0.001 M EDTA. The size of the DNA was estimated to be greater than 24 kbp in length.

Example 2    Growth of Bacteriophage

Both the Charon 28 (Rimm, D.L., Horness, D., Kucera, J., and Blattner, F.R. (1980) Gene 12, 301-309) and recombinant phage were prepared using a liquid lysis technique. Approximately $4 \times 10^9$ phage were preadsorbed to $2 \times 10^{10}$ cells (K802) in 5 ml of SM buffer (0.1 M NaCl, 0.01 M MgSO$_4$, 0.05 M Tris-HCl, pH 7.5, 0.01% gelatin) at 37°C for 15 minutes. The phage-cell mixture was then added to 500 ml of NZCYM broth (10 g NZamine, 5 g NaCl, 5 g yeast extract, 1 g casamino acids, 10 ml 1 M MgSO$_4$ per liter; pH to 7.5 with NaOH) and was vigorously agitated at 37°C for 12 hours. 1.5 ml of CHCl$_3$ was then added, followed by centrifugation for 10 mintues at 5000 rpm to remove any cells and debris. After dissolving 50 g of polyethylene glycol 6000 (Sigma) and 29.2 g of NaCl in the supernatant, the sample was incubated 2-3 hours at 4° and then centrifuged at 8000 rpm for 10 minutes. The phage pellet was redissolved in 5 ml of SM buffer. Cesium chloride was added (0.75 g per ml volume) and the sample was centrifuged to equilibrium in an SW41 rotor at 28,000 rpm. The phage band was removed through the side of the tube with a 22 guage needle and dialyzed in 0.01 M tris-HCl, pH 7.5, 0.001 M EDTA.

Example 3    Extraction of Bacteriophage DNA

SDS and EDTA were added to an aliquot of the phage suspension to final concentrations of 0.1% and 0.002 M respectively. After heating for 15 minutes at 65°C, the sample was extracted once with an equal volume of Tris-saturated phenol followed by two chloroform extractions. The DNA was then purified with a single ethanol precipitation.

Example 4    Preparation of Charon 28 Arms

Charon 28 DNA was digested with Bam HI (1 unit of enzyme per µg of DNA), then placed on a 38.5 ml 10-40% sucrose gradient (1 M NaCl, 0.01 M Tris-HCl, pH 8). The gradient was centrifuged for 24 hours at 26,000 rpm in an SW 25 rotor. Fractions containing the arms (identified by agarose gel electrophoreses) were pooled, dialyzed against 0.01 M Tris-HCl, pH 8, 0.0005 M EDTA and were then concentrated to 0.5 ml with sec-butanol. After ethanol precipitation, the sample was stored in water at -80°C.

Example 5    Preparation of Bovine Insert DNA for Cloning

Genomic placental DNA was partially digested with the restriction enzyme Mbo I. The resulting fragments were size fractionated on a 10-40% sucrose gradient (1M NaCl, 0.005 M EDTA). Fractions containing 15-18 kbp fragments were pooled and ethanol precipitated. The DNA was resuspended in water and stored at -80°C.

### Example 6    Preparation of λ Packaging Extracts

The two complementary λ lysogenic bacterial strains, BHB 2688 and BHB 2690, as well as the procedure used is described in Hohn, B. (1979) in Methods in Enzymology (R. Wu ed.) Vol. 68, pp. 229-309, Academic Press, New York.

### A. Sonic Extracts

A 500 ml culture of BHB 2690 was grown to an $A_{600}$ = 0.3 at 32°C in NZ broth (10 g NZamine, 5 g NaCl, 2 g $MgCl_2 \cdot 6H_2O$ per liter, pH 7.5). The endogenous phage was induced by heating at 45°C for 15 minutes and was then further incubated for 3 hours at 37°C. The cells were pelleted by centrifugation, then resuspended in 3.6 ml of sonication buffer (0.02 M Tris-HCl, pH 8, 0.001 M EDTA, 0.003 M $MgCl_2$, 0.005 M β-mercaptoethanol). The sample was pulse sonicated until clear, then centrifuged for 10 minutes at 10,000 rpm. An equal volume of cold sonication buffer and 1/6 volume of packaging buffer (0.006 M Tris-HCl, pH 8, 0.05 M spermidine, 0.05 M putrescine, 0.02 M $MgCl_2$, 0.02 M ATP and 0.03 M β-mercaptoethanol) were added to the supernatant. The solution was frozen in liquid nitrogen in 15 μl aliquots.

### B. Freeze-thaw Lysates

A 1500 ml culture of BHB 2688 was grown and induced according to the procedure described above for BHB2690. The cells were then pelleted and resuspended in 3 ml of 10% sucrose, 0.05 M Tris-HCl, pH 7.5 and 0.5 ml aliquots of the solution were distributed among six 1.5 ml eppendorf tubes. To each tube, 25 μl of a 2 mg/ml lysozyme in 0.25 M Tris-HCl, pH 7.5 solution was added. After mixing well, the solutions were frozen in liquid nitrogen and allowed to thaw on ice. Packaging buffer (25 μl) was added to each tube before centrifugation for 1 hour at 20,000 rpm. The supernatant was frozen in liquid nitrogen in 10 μl aliquots.

### Example 7    Ligation of the Bovine Insert DNA to the Charon 28 Arms and In Vitro Packaging

A 2:1 molar ratio of arms:insert was empirically determined to give the best results. 3.1 μg of λ arms DNA and 0.9 μg of bovine

insert DNA were combined and ligated together with T4 DNA ligase at 16°C for 4-6 hours in 0.01 M Tris-HCl, pH 7.5, 0.01 M $MgCl_2$, 0.006 M KCl, 0.001 M ATP and 0.01 M dithiothritol. The DNA was packaged into λ capsids *in vitro* by incubating (60 minutes at room temperature) 1-5μl of the ligated DNA sample with 15 μl of the sonic extract and 10μl of the freeze-thaw lysate. A total of $2 \times 10^6$ pfu's (plaque-forming units) were generated in this manner.

Example 8    Library Amplification and Screening

A total of 900,000 pfu's were amplified according to the plate lysate technique described by Enquist and Sternberg (Enquist, L., and Sternberg, N. (1979) in Methods in Enzymology (R. Wu ed.) Vol. 68, pp. 229-309, Academic Press, New York).

Approximately 700,000 pfu's from the amplified library were screened using the Benton and Davis method (Benton, W.D., and Davis, R. (1977) Science 196, 180-182). To do this, recombinant phage were initially plated at a very high density (300 plaques per $cm^2$) on large NZCYM plates (10 g NZamine, 5 g yeast extract, 1 g casamino acids, 10 ml 1 M $MgSO_4$ and 15 g bactoagar per liter, pH 7.5). Duplicate impressions were made of the agarose surface on two Millipore nitrocellulose filters. The nitrocellulose filters were then processed as follows: 1 minute in 0.5 M NaOH, 1.5 M NaCl; 5 minutes in 1.5 M NaCl, 0.5 M Tris-HCl, pH 8; 1 minute in 0.18 M NaCl, 0.01 M $Na_2HPO_4$, 0.001 M EDTA. The phage DNA was fixed to the nitrocellulose by heating the filters for 2 hours at 80°C in a vacuum oven. After the filters were prewashed for 60 minutes at 42°C in 1 M NaCl, 0.05 M Tris-HCl, pH 8, 0.001 M EDTA and 0.1% SDS, they were prehybridized for at least 4 hours at 42°C in 50% formamide, 5XSSPE (1XSSPE is 0.09 M NaCl, 0.005 M $Na_2HPO_4$ and 0.005 M EDTA, pH 7), 1X Denhardt's solution (0.02% polyvinyl pyrollidone 0.02% bovine serum albumin, 0.02% ficoll) and 100 μg/ml denatured salmon sperm DNA. Nick-translated insert DNA from a bovine prolactin cDNA clone and a growth hormone cDNA clone was then added directly to the prehybridization solution (approximately $1.5 \times 10^5$ cpm/ml of each insert) and was allowed to hybridize for 36 hours at 42°C. The filters were washed according to the following sequence: 4 washes in 2XSSC-0.1% SDS, 23°C, 15 minutes each; one wash in 1XSSC-0.1% SDS, 30 minutes, 65°C; 2 washes in 0.2XSSC-0.1% SDS, 45-60 minutes, 65°C. After drying the filters they were placed against XAR-5 X-ray film, sandwiched between two lightning-plus intensifying screens and stored at -80°C. Positive signals that were present at the same position on both filters were further screened at lower plating densities and were ultimately plaque purified.

Example 9 . Subcloning of Restriction Fragments into pBR322

The technique used for subcloning of the 4.3 kbp EcoRI restriction fragment from the genomic clone λGh2 into pBR322 was similar to that described in Yen, P., and Davidson, N. (1980) Cell 22, 137-148. Briefly, λGh2 DNA was digested with EcoRI, heated to 65°C for 10 minutes to inactivate the enzyme and then ethanol precipitated. pBR322 was digested with EcoRI, subjected to bacterial alkaline phosphatase treatment, phenol extracted and then ethanol precipitated. Aliquots of the digested λGh2 pBR322 were combined and ligated together with T4 DNA ligase in 0.01 M Tris-HCl, pH 7.5, 0.01 M MgCl$_2$, 0.006 M KCl, 0.001 M ATP and 0.01 M dithiothreitol. The ligated material was then used directly to transform E. coli HB101 (Villa-Komaroff, L., Efstratiadis, A., Broome, S., Lomedico, P., Tizard, R., Naber, S.P., Chick, W.L., and Gilbert, W. (1978) Proc. Natl. Acad. Sci. USA 75, 3727-3731). The colony screening technique described by Grunstein and Hogness (Grunstein, M., and Hogness, D.S. (1975) Proc. Natl. Acad. Sci. USA 72, 3961-3965) was used to positively select for the desired clones.

Example 10 DNA Sequence Analysis

The dideoxy chain terminator sequencing technique (Sanger, F., Nicklen, S., and Coulson, A. R. (1977) Proc. Natl. Acad. Sci. USA 74, 5463-5467) was used to sequence the entire bovine growth hormone gene. Deoxyinosine 5' triphosphate (Sigma) was used in some dideoxy sequencing reactions instead of deoxyguanosine 5' triphosphate in order to resolve some compressed areas on the sequence. Sequencing templates were prepared by subcloning restriction fragments of the genomic sequence into the BamHI, PstI and SmaI sites of the M13 cloning vector mp8. The universal sequencing primer (P-L Biochemicals) was used in the individual reactions.

0112012

## CLAIMS

1. The nucleotide sequence of genomic bovine growth hormone as follows:

```
AAAACCTATGGGGTGGGCTCTCAAGCTGAGACCCTGTGTGCACAGCCCTCTGGCTGGTGGCAGTGGAG

ACGGGATNNNATGACAAGCCTGGGGGACATGACCCCAGAGAAGGAACGGGAACAGGATGAGTGAGAGGAG

GTTCTAAATTATCCATTAGCACAGGCTGCCAGTGGTCCTTGCATAAATGTATAGAGCACACAGGTGGGGG

                                                                    5'
GAAAGGGAGAGAGAGAAGAAGCCAGGGTATAAAAATGGCCCAGCAGGGACCAATTCCAGGATCCCAGGAC
                                                        -26
                                                        Met Met Ala Ala
CCAGTTCACCAGACGACTCAGGGTCCTGTGGACAGCTCACCAGCT    ATG ATG GCT GCA G gtaag

ctcgctaaaatcccctccattcgcgtgtcctaaaggggtaatgcggggggccctgccgatggatgtgttc

agagctttgggctttagggcttccgaatgtgaacataggtatctacacccagacatttggccaagtttga

aatgttctcagtccctggagggaagggtaggtgggggctggcaggagatcaggcgtctagctccctgggg
                                                -20
                                                Gly Pro Arg Thr Ser Leu Leu Leu Ala
ccctccgtcgcggccctcctggtctctccctag    GC CCC CGG ACC TCC CTG CTC CTG GCT

          -10                                              1
Phe Ala Leu Leu Cys Leu Pro Trp Thr Gln Val Val Gly Ala Phe Pro Ala
TTC GCC CTG CTC TGC CTG CCC TGG ACT CAG GTG GTG GGC GCC TTC CCA GCC

                    10                                          20
Met Ser Leu Ser Gly Leu Phe Ala Asn Ala Val Leu Arg Ala Gln His Leu
ATG TCC TTG TCC GGC CTG TTT GCC AAC GCT GTG CTC CGG GCT CAG CAC CTG

                              30
His Gln Leu Ala Ala Asp Thr Phe Lys Glu Phe
CAT CAG CTG GCT GCT GAC ACC TTC AAA GAG TTT    gtaagctcccgagggatgcgt

cctaggggtggggaggcaggaaggggtgaatccacaccccctccacacagtgggaggaaactgaggagtt

cagccgtattttatccaagtagggatgtggttaggggagcagaaacggggggtgtgtggggtggggagggt

tccgaataaggcggggagggggaaccgcgcaccagcttagacctgggtgggtgtgttcttcccccag

                              40
Glu Arg Thr Tyr Ile Pro Glu Gly Gln Arg Tyr Ser Ile Gln Asn Thr Gln
GAG CGC ACC TAC ATC CCG GAG GGA CAG AGA TAC TCC ATC CAG AAC ACC CAG

- 50                                          60
Val Ala Phe Cys Phe Ser Glu Thr Ile Pro Ala Pro Thr Gly Lys Asn Glu
GTT GCC TTC TGC TTC TCT GAA ACC ATC CCG GCC CCC ACG GGC AAG AAT GAG
```

```
                    70
Ala Gln Gln Lys Ser
GCC CAG CAG AAA TCA    gtgagtggcaacctcggaccgaggagcaggggacctccttcatcctaa

gtaggctgccccagctctccgcaccgggcctggggcggccttctccccgaggtggcggaggttgttggat

ggcagtggaggatgatggtgggcggtggtggcaggaggtcctcgggcagaggccgaccttgcagggctgc

                                               Asp Leu Glu Leu Leu Arg Ile
cccaagcccgcggcacccaccgaccacccatctgccagcag   GAC TTG GAG CTG CTT CGC ATC

    80                                               90
Ser Leu Leu Leu Ile Gln Ser Trp Leu Gly Pro Leu Gln Phe Leu Ser Arg
TCA CTG CTC CTC ATC CAG TCG TGG CTT GGG CCC CTG CAG TTC CTC AGC AGA

            100                                          110
Val Phe Thr Asn Ser Leu Val Phe Gly Thr Ser Asp Arg Val Tyr Glu Lys
GTC TTC ACC AAC AGC TTG GTG TTT GGC ACC TCG GAC CGT GTC TAT GAG AAG

                            120
Leu Lys Asp Leu Glu Glu Gly Ile Leu Ala Leu Met Arg
CTG AAG GAC CTG GAG GAA GGC ATC CTG GCC CTG ATG CGG gtggggatggcgt

tgtgggtcccttccatgctggggggccatgcccgccctctcctggcttagccaggagaatgcacgt

gggcttggggagacagatccctgctctctccctctttctagcagtccagccttgacccagggggaa

acctttcccctttttgaaacctccttcctcgcccttctccaagcctgtaggggagggtggaaaat

ggagcgggcaggagggagctgctcctgagggcccttcggcctctctgtctctccctcccttggcag

            130                                          140
Glu Leu Glu Asp Gly Thr Pro Arg Ala Gly Gln Ile Leu Lys Gln Thr
GAG CTG GAA GAT GGC ACC CCC CGG GCT GGG CAG ATC CTC AAG CAG ACC

                            150
Tyr Asp Lys Phe Asp Thr Asn Met Arg Ser Asp Asp Ala Leu Leu Lys
TAT GAC AAA TTT GAC ACA AAC ATG CGC AGT GAC GAC GCG CTG CTC AAG

    160                                          170
Asn Tyr Gly Leu Leu Ser Cys Phe Arg Lys Asp Leu His Lys Thr Glu
AAC TAC GGT CTG CTC TCC TGC TTC CGG AAG GAC CTG CAT AAG ACG GAG

                    180                          190
Thr Tyr Leu Arg Val Met Lys Cys Arg Arg Phe Gly Glu Ala Ser Cys
ACG TAC CTG AGG GTC ATG AAG TGC CGC CGC TTC GGG GAG GCC AGC TGT

Ala Phe am
GCC TTC TAG    TTGCCAGCCATCTGTTGTTTGCCCCTCCCCCGTGCCTTCCTTGACCCTG

                                               ↓3'
GAAGGTGCCACTCCCACTGTCCTTTCCTAATAAAATGAGGAAATTGCATCGC ATTGTCTG

AGTAGGTGTCATTCTATTCTGGGGGGGTGGGGTGGGGCAGGACAGCAAGGGGGAGGATTGGGA

AGACAATAGCAGGCATGCTGGGGATGCGGTGGGCTCTATGGGTACCCAGGTGCTGAAGAA
```

12

TTGACCCGGTTCCTCCTGGG.

2.    A DNA transfer vector comprising the nucleotide sequence identified in claim 1 or a functional equivalent thereof.

3.    A host modified to contain the nucleotide sequence identified in claim 1 or a functional equivalent thereof.

4.    A host according to claim 3, which is a micro-organism containing a replicatable DNA transfer vector according to claim 2.

5.    A host according to claim 4, in which the micro-organism is <u>Escherichia coli</u> X 1776 or <u>Escherichia coli</u> HB101, and in which the plasmid is pBR322.

6.    A process for preparing bovine growth hormone, which comprises culturing a host according to any of claims 3 to 5.

7.    Plasmid pλGH2R2.

8.    Plasmid pλGH2R3.

9.    <u>E. coli</u> HB101 (pλGH2R2), NRRL B-15154.

10.    <u>E. coli</u> HB101 (pλGH2R3), NRRL B-15155.

FIGURE I

FIGURE 2